# EUROPEAN PATENT SPECIFICATION

(11) **EP 1 631 213 B1**
(45) Date of publication and mention of the grant of the patent: **04.12.2013**
(21) Application number: 04734483.3
(22) Date of filing: 23.05.2004
(51) Int. Cl.: A61F 2/958

(54) **STENT POSITIONING SYSTEM**
STENTPOSITIONIERUNGSSYSTEM
SYSTEME DE MISE EN PLACE D'UNE ENDOPROTHESE

(30) Priority: 26.05.2003 IL 15611503
(43) Date of publication of application: 08.03.2006
(73) Proprietor: HADASIT MEDICAL RESEARCH SERVICES & DEVELOPMENT CO. LTD., Jerusalem 91120 (IL)
(72) Inventor: VARSHITZKY, Boris, IL-93840 Jerusalem (IL); BORIVKER, Nachum, 93898 Jerusalem (IL); LOTAN, Chaim, IL-96263 Jerusalem (IL); MOSSERI, Moriss, IL-96782 Jerusalem (IL)
(74) Representative: Petruzziello, Aldo
(86) International application number: PCT/IL2004/000436
(87) International publication number: WO 2004/103216

(56) References cited:
- WO-A-99/36015
- WO-A-03/105695
- US-A- 5 749 890
- US-A1- 2002 091 434

## Description

### Field of the Invention

The present invention relates to a stent positioning system, more specifically, to such a system for the treatment of aorto-ostial lesions.

### Background of the Invention

An ostial lesion is defined as one arising within 3 mm of the origin of a blood vessel. Aorto-ostial lesions are those which damage the ostium of the main blood vessels branching from the aorta.

The treatment of aorto-ostial lesions by conventional balloon angioplasty has shown a low success rate and a high incidence of re-stenosis. An attractive alternative for the treatment of this subset of lesions is coronary stenting.

A serious difficulty in the implantation of a stent in an aorto-ostial location is the determination of the exact position where the stent is to be disposed. If the stent is placed too far inside the vessel, it misses the ostium and the tightest portion of the stenosis. Yet, if the stent is placed too proximally, it extends into the aorta and may be subject to trauma from the guiding catheter. The potentials of compromising the aortal lumen and increasing the dangers of stent thrombosis and re-stenosis, also exist.

U.S. Patent Application 2002/0091434 A1 discloses an apparatus and method for positioning a stent, where the positional element is constituted by wire loops for frictional engagement with the walls to position the stent at the deployment site. Upon exiting the guiding catheter, the wire loops automatically expand and engage the artery walls.

US Pat. No. 5,749,890 (Shaknovich) entitled *"Method and system for stent placement in ostial lesions"* discloses a stent delivery assembly and method for stent placement in an ostial lesion. In particular, the stent delivery system disclosed thereby comprises a break segment, which changes configuration to facilitate localization of the target ostium.
WO9936015 entitled *"Catheter and method of ostial stent placement"* discloses a catheter with two balloons for placing a stent at the ostium or junction of a variety of bodily tissues, including the junction of any artery, and an aorta to afford improved treatment of stenotic lesions.

WO 03/105695 **discloses a stent comprising an expandable cylindrical section and a flaring section attached thereto at a plurality of fingers, wherein the fingers couple radial expansion of the cylindrical section with flaring of the flaring section.**

### Disclosure of the Invention

It is thus one of the objects of the present invention to provide a stent delivery system that prevents both too distal and too proximal placement and implantation of the stent, i.e., a system that ensures proper stent-to-vessel apposition.
According to the invention, the above object is achieved by providing a stent positioning system according to claims 1, **4 and 6.**

### Brief Description of the Drawings

The invention will now be described in connection with certain preferred embodiments with reference to the following illustrative figures, so that it may be more fully understood.

With specific reference now to the figures in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
- Fig. 1: illustrates a typical aorto-ostial lesion;
- Figs. 2 and 3: illustrate two types of faulty prior art stent-to-vessel apposition;
- Figs. 4 and 5: illustrate a preferred embodiment of the stent positioning system according to the present invention, in two different stages of use;
- Fig. 6: is a cross-sectional view of the stent positioning system of Fig. 5, in a third stage of use;
- Fig. 7: is a cross-sectional view along line VII-VII of Fig. 6;
- Figs. 8 to 10: illustrate a further embodiment of the stent positioning system according to the present invention, in three different stages of use;
- Figs. 11 to 13: illustrate still a further embodiment of the stent positioning system according to the present invention, in three different stages of use;
- Fig. 14: is a cross-sectional view along line XIV-XIV of Fig. 13;
- Figs. 15 and 16: illustrate still a further embodiment of the stent positioning system according to the present invention, in two different stages of use;
- Fig. 17: is a cross-sectional view of the stent positioning system of Fig. 16, in a third stage of use, and
- Fig. 18: is a cross-sectional view along line XVIII-XVIII of Fig. 17.

### Detailed Description of Preferred Embodiments

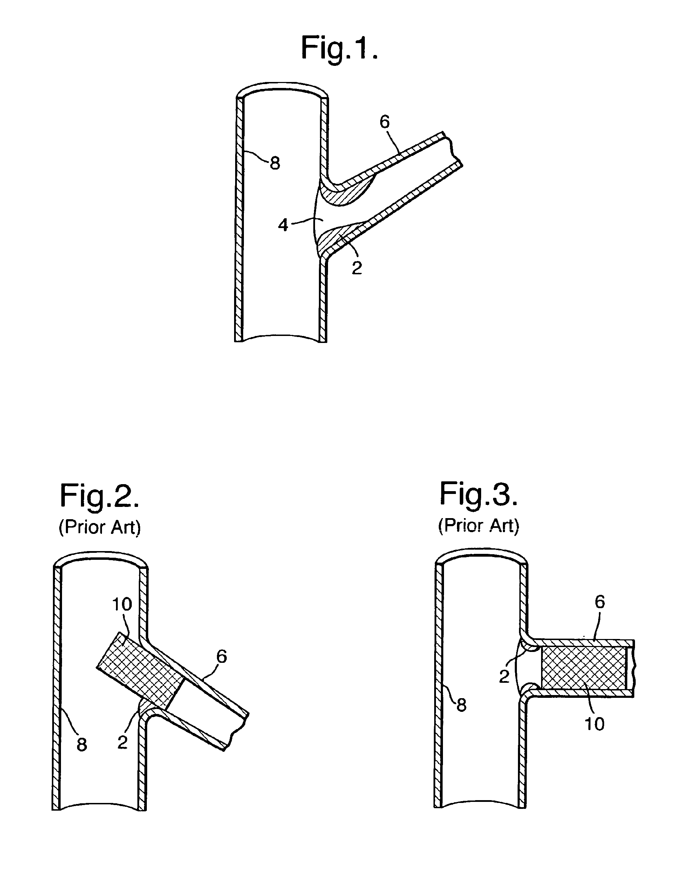

Referring now to the drawings, there is illustrated in Fig. 1 a typical ostial lesion, defined as a lesion arising within 3 mm of the origin of the blood vessel. More specifically, Fig. 1 illustrates an aorto-ostial atherosclerotic lesion, that produces a significant stenosis 2 at the ostium 4 of a coronary artery 6, where the artery branches off the aorta 8.

Figs. 2 and 3 illustrate faulty prior art stent-to-vessel apposition. In Fig. 2, stent 10 is implanted in too proximal a location and is seen to project into aorta 8, where it is subject to trauma from the guiding catheter and is also liable to compromise the lumen of the aorta, increasing the danger of stent thrombosis and re-stenosis. In Fig. 3, the stent is placed in too distal a location, missing the ostium 2 and the tightest portion of the stenosis.

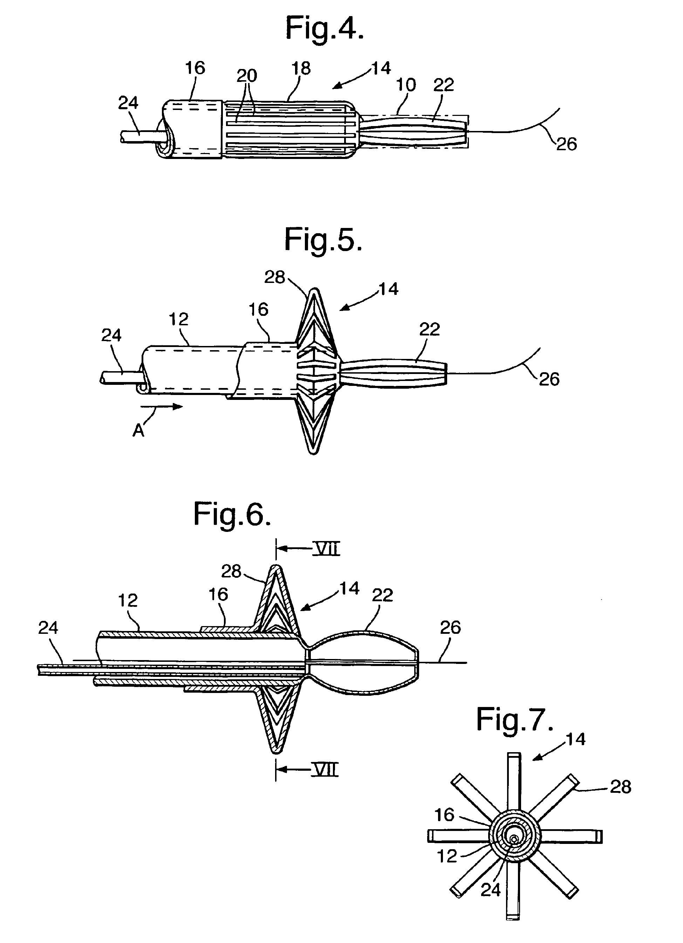

The above-mentioned mishaps are avoided by the use of the stent positioning system of the present invention, a preferred embodiment of which is shown in Figs. 4 to 7. The system is seen to consist of a guide tube 12, a locator 14, a sleeve having a first portion constituting an actuator 16, and a second portion 18 made of an elastomer. Portion 18 is provided with slots 20 extending in the longitudinal direction of the sleeve 18, possibly around its entire periphery. The system further includes a stent-expansion balloon 22 attached to the guide tube 12 and an inflation tube 24 through which balloon 22 can be inflated in order to expand the stent 10, indicated by dash-dotted lines in Fig. 4. Also seen in the Figures is a thin special wire 26 passing through the system for the purpose, per-se known. When the actuator 16 is pushed in the direction of arrow A (Fig. 5), the elastomer portion 18 of the sleeve will buckle and assume a substantially disk-like shape while forming a locator 14 having a plurality of fingers 28 in a star-like configuration (Fig. 7). Once the balloon 22 is accurately positioned in place by means of the locator 14, it is inflated (see Fig. 6), thereby expanding the stent 10. The remaining procedure is self-evident by men skilled in the art.

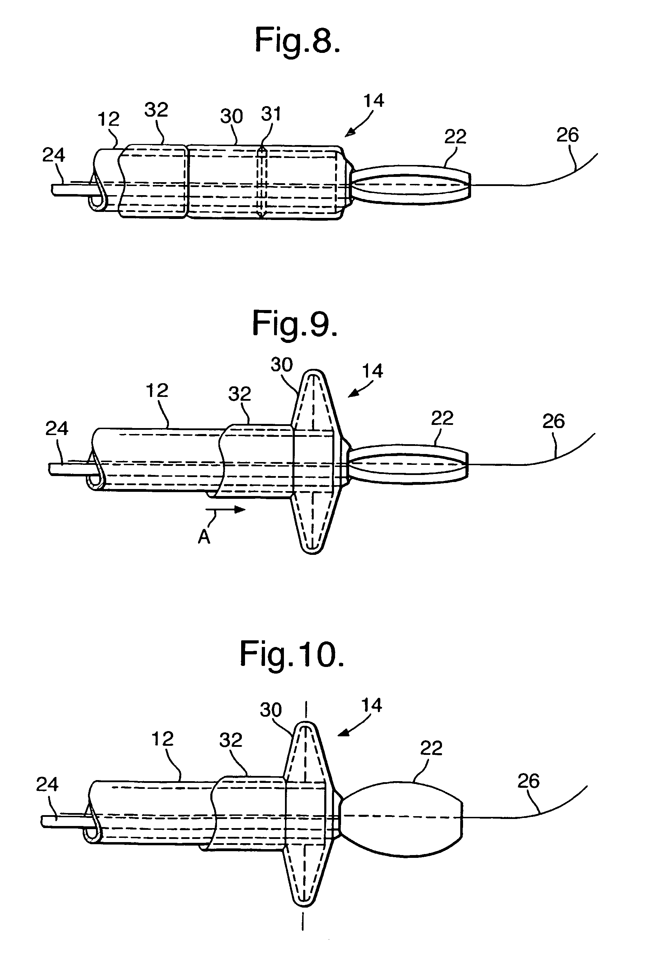

A variation of the above-described preferred embodiment is seen in Figs. 8 to 10. In this embodiment the stent locator is in the form of a two-portion sleeve 30 made of an elastomer, which portions are advantageously separated by a weakened cross-section 31. The expansion balloon side of sleeve 30 is fixedly attached to the guide tube 12 and the other end of the sleeve 30 is attached to an actuator 32 in the form of a tube. When actuator 32 is pushed relative to guide tube 12 in the direction of arrow A, the elastomer sleeve 30 will buckle about its weakened cross-section 31, and assume a substantially disk-like shape, adapted to act as a stent locator 14 (Fig. 9). When the stent is in position, the balloon 22 can be inflated (Fig. 10) to expand the stent.

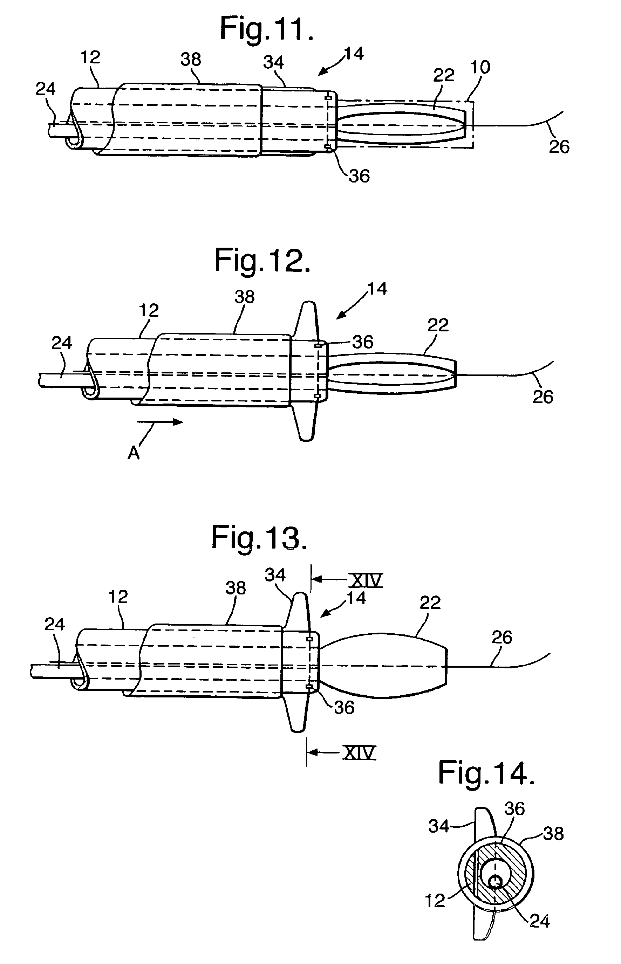

Turning now to Figs. 11 to 14, there is illustrated a stent locator 14 in the form of a piece of wire 34, shown in Fig. 11 in its collapsed state. The piece of wire 34 is substantially U-shaped in its state of rest. The web of this U-shape passes through lateral holes 36 in the guide tube 12 and the two legs of the U are fixedly attached to the actuator 38 surrounding guide tube 12. When actuator 38 is pushed relative to guide tube 12 in the direction of arrow A (Fig. 12), the U-shaped wire 34 is elastically deformed, as shown in Figs. 12 to 14, increasing the length of the web, which can now act as a stent locator 14.

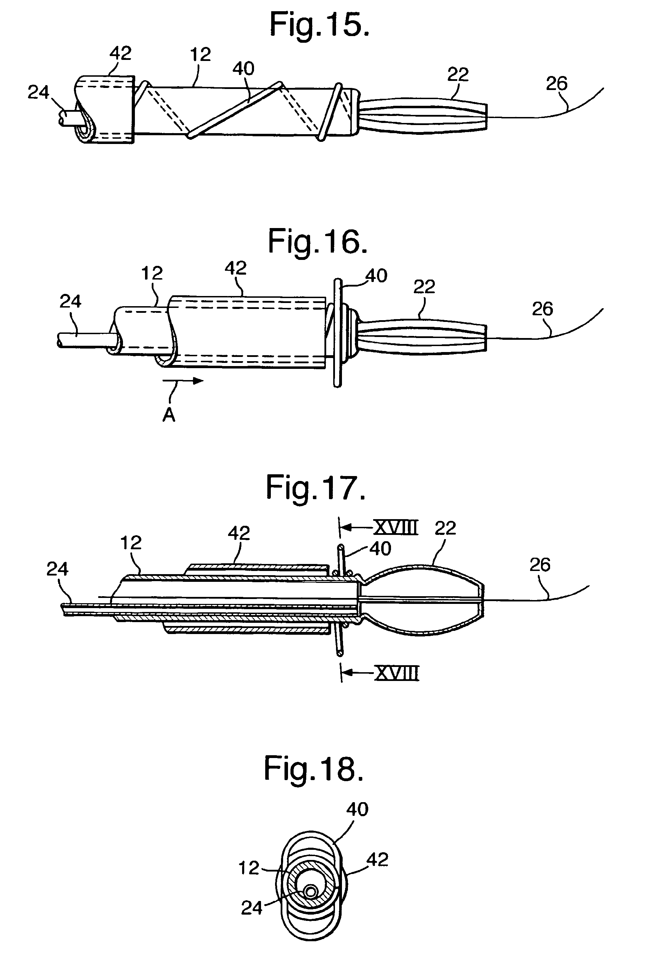

A further embodiment is illustrated in Figs. 15 to 18. Accordingly, the sleeve 18 of Fig. 4 and sleeve 30 of Fig. 8 is replaced by a spring element 40 wound about the guide tube 12. The ends of the spring element 40 respectively, abut against, or are attached to, an actuator 42 and the end of the guide tube 12 close to the expansion balloon 22. Upon sliding the actuator 42 in the direction of arrow A towards the expansion balloon 22 (Fig. 16), the spring element 40 is compressed and forms loops (Fig. 18), extending laterally from the axis of the expansion balloon 20 and stent (not shown) positioned thereon, so as to constitute an abutment for accurately locating the expandable stent mounted on the expansion balloon 22.

The described stent positioning systems may be used for accurate stent implantation in any and all possible stenting locations, e.g., the aorta and all possible small or large branches arising from the aorta (coronary, carotid, subclavian, mesenteric, renal, iliac and other arteries), central and peripheral vein system (porto-caval stent etc.), biliary system, and tracheal location.

It will be evident to those skilled in the art that the invention is not limited to the details of the foregoing illustrated embodiments. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein.

## Claims

1. A stent positioning system, comprising:
an inflatable balloon (22) for expanding a stent, said balloon, in its collapsed state, fitting into and being adapted to carry said stent in its pre-expanded condition;
stent locator means (14) adapted for sliding accommodation in a guide catheter and adapted to change its shape prior to making contact with the interior wall surface of a major blood vessel in the ostial region of a smaller blood vessel branching off from said major vessel and prior to the expansion of said stent, and
mechanical means (16) for changing the shape of said stent locator means;
**characterized in that**:
the change of shape enables said locator means to abut said interior wall surface, thereby ensuring correct apposition between the stent and the ostium of said smaller blood vessel;
the mechanical means is constituted by an actuator tube (16) surrounding a guide tube (12); and
the stent locator means is in the form of a sleeve (18) made of an elastomer, the expansion balloon side of said sleeve being fixedly attached to the guide tube (12) and the other end of said sleeve being fixedly attached to the actuator tube (16).

2. The system as claimed in claim 1, wherein said elastomer sleeve is provided with a plurality of longitudinal slots (20), wherein, when said actuator tube is pushed relative to said guide tube in the direction of said expansion balloon, the elastomer sleeve will buckle and form a plurality of fingers.

3. The system as claimed in claim 1, wherein said elastomer sleeve is provided with a weakened cross-section (31) at about half its length, said weakened cross-section facilitating the buckling of said sleeve (30).

4. A stent positioning system, comprising:
an inflatable balloon (22) for expanding a stent, said balloon, in its collapsed state, fitting into and being adapted to carry said stent in its pre-expanded condition;
stent locator means (14) adapted for sliding accommodation in a guide catheter and adapted to change its shape prior to making contact with the interior wall surface of a major blood vessel in the ostial region of a smaller blood vessel branching off from said major vessel and prior to the expansion of said stent; and
mechanical means (32) for changing the shape of said stent locator means;
**characterized in that**:
the change of shape enables said locator means to abut said interior wall surface, thereby ensuring correct apposition between the stent and the ostium of said smaller blood vessel;
the mechanical means is constituted by an actuator tube (32) surrounding a guide tube (12); and
the stent locator means, in its state of rest, is in the form of a substantially U-shaped piece of wire, having a web and two legs, wherein, when said actuator tube is pushed relative to the guide tube in the direction of the inflatable balloon for the expansion of said stent, the U-shaped piece of wire is elastically deformed, substantially increasing the length of said web and thereby rendering it capable of abutting the interior wall surface of said major blood vessel.

5. The system as claimed in claim 4, wherein said web passes through a lateral hole (36) in the guide tube (12) and the two legs of which are each fixedly attached to an actuator tube (16) surrounding said guide tube (12).

6. A stent positioning system, comprising:
an inflatable balloon (22) for expanding a stent, said balloon, in its collapsed state, fitting into and being adapted to carry said stent in its pre-expanded condition;
stent locator means (14) adapted for sliding accommodation in a guide catheter and adapted to change its shape prior to making contact with the interior wall surface of a major blood vessel in the ostial region of a smaller blood vessel branching off from said major vessel and prior to the expansion of said stent; and
mechanical means (42) for changing the shape of said stent locator means;
**characterized in that**:
the change of shape enables said locator means to abut said interior wall surface, thereby ensuring correct apposition between the stent and the ostium of said smaller blood vessel;
the mechanical means is constituted by an actuator tube (42) surrounding a guide tube (12); and
the stent locator means is a spring element (40) wound about the tube (12) and having opposing ends that respectively, abut against, or are attached to, the actuator tube (42) and the end of the tube (12) close to the balloon (22);
whereupon sliding the actuator (42) towards the expansion balloon (22) compresses the spring element (40) and forms loops that extend laterally from an axis of the balloon (20) and stent positioned thereon, so as to constitute an abutment for accurately locating the expandable stent mounted on the balloon.

## Patentansprüche

1. Stentpositionierungssystem, umfassend
einen inflatierbaren Ballon (22) zum Ausdehnen eines Stents, wobei der Ballon in seinem zusammengefalteten Zustand in den Stent passt und eingerichtet ist, den Stent in seinem Status vor der Ausdehnung zu transportieren;
Lokalisierungsmittel des Stents (14), die zur gleitenden Unterbringung in einem Führungskatheter eingerichtet sind, und die eingerichtet sind, ihre Gestalt vor der Berührung der Innenwandoberfläche eines großen Blutgefäßes in dem ostialen Bereich eines kleineren Blutgefäßes, das von dem großen Blutgefäß abzweigt, und vor der Ausdehnung des Stents zu verändern, und
mechanische Mittel (16) zum Verändern der Gestalt der Lokalisierungsmittel des Stents;
**dadurch gekennzeichnet, dass**
die Veränderung der Gestalt die Lokalisierungsmittel in die Lage versetzt, an die Innenwandoberfläche anzustoßen, wodurch die korrekte Anlagerung zwischen dem Stent und dem Ostium des kleineren Blutgefäßes sichergestellt wird;
die mechanischen Mittel aus einem Betätigungsschlauch (16) bestehen, der einen Führungsschlauch (12) umschließt; und
die Lokalisierungsmittel des Stents sich in Gestalt einer Umhüllung (18) darstellen, die aus Elastomer besteht, wobei die Ballon-Ausdehnungsseite der Umhüllung fest mit dem Führungsschlauch (12) verbunden ist und das andere Ende der Umhüllung fest mit dem Betätigungsschlauch (16) verbunden ist.

2. System nach Anspruch 1, wobei die Elastomerumhüllung mit einer Vielzahl von länglichen Schlitzen (20) versehen ist, wobei, wenn der Betätigungsschlauch im Verhältnis zu dem Führungsschlauch in Richtung des Ausdehnungsballons gedrückt wird, sich die Elastomerumhüllung auswölben und eine Vielzahl von Lamellen ausbilden wird.

3. System nach Anspruch 1, wobei die Elastomerumhüllung ungefähr an der Hälfte ihrer Länge mit einem abgeschwächten Querschnitt (31) versehen ist, wobei der abgeschwächte Querschnitt das Auswölben der Umhüllung (30) erleichtert.

4. Stentpositionierungssystem, umfassend
einen inflatierbaren Ballon (22) zum Ausdehnen eines Stents, wobei der Ballon, in seinem zusammengefalteten Zustand, in den Stent passt und eingerichtet ist, den Stent in dessen Status vor der Ausdehnung zu transportieren;
Lokalisierungsmittel des Stents (14), die zur gleitenden Unterbringung in einem Führungskatheter eingerichtet sind, und die eingerichtet sind, ihre Gestalt vor der Berührung der Innenwandoberfläche eines großen Blutgefäßes in dem ostialen Bereich eines kleineren Blutgefäßes zu verändern, das von dem großen Blutgefäß abzweigt, und vor der Ausdehnung des Stents; und
mechanische Mittel (32) zum Verändern der Gestalt der Lokalisierungsmittel des Stents;
**dadurch gekennzeichnet, dass**
die Veränderung der Gestalt die Lokalisierungsmittel in die Lage versetzt, an die Innenwandoberfläche anzustoßen, wodurch die korrekte Anlagerung zwischen dem Stent und dem Ostium des kleineren Blutgefäßes sichergestellt wird;
die mechanischen Mittel aus einem Betätigungsschlauch (32) bestehen, der einen Führungsschlauch (12) umschließt; und
die Lokalisierungsmittel des Stents, in ihrem Ruhezustand, sich in der Gestalt eines im Wesentlichen U-förmigen Drahtstückes darstellen, das einen Steg und zwei Füße aufweist, wobei, wenn der Betätigungsschlauch im Verhältnis zu dem Führungsschlauch in Richtung des inflatierbaren Ballons zur Ausdehnung des Stents geschoben wird, das U-förmige Drahtstück elastisch verformt wird, wobei im Wesentlichen die Länge des Steges verlängert wird und es dadurch in die Lage versetzt wird, an die Innenwandoberfläche des großen Blutgefäßes anzustoßen.

5. System nach Anspruch 4, wobei der Steg durch eine laterale Öffnung (36) in dem Führungsschlauch (12) hindurchgeht und wobei dessen beide Füße fest an einem Betätigungsschlauch (16) befestigt sind, der den Führungsschlauch (12) umschließt.

6. Stentpositionierungssystem, umfassend
einen inflatierbaren Ballon (22) zum Ausdehnen eines Stents, wobei der Ballon in seinem zusammengefalteten Zustand in den Stent passt und eingerichtet ist, den Stent in seinem Status vor der Ausdehnung zu transportieren;
Lokalisierungsmittel des Stents (14), die zur gleitenden Unterbringung in einem Führungskatheter eingerichtet sind, und die eingerichtet sind, ihre Gestalt vor der Berührung einer Innenwandoberfläche eines großen Blutgefäßes in dem ostialen Bereich eines kleineren Blutgefäßes zu verändern, das von dem großen Blutgefäß abzweigt, und vor der Ausdehnung des Stents; und
mechanische Mittel (42) zum Verändern der Gestalt der Lokalisierungsmittel des Stents;
**dadurch gekennzeichnet, dass**
die Veränderung der Gestalt die Lokalisierungsmittel in die Lage versetzt, an die Innenwandoberfläche anzustoßen, wobei die korrekte Anlagerung zwischen dem Stent und dem Ostium des kleineren Blutgefäßes sichergestellt wird;
die mechanischen Mittel aus einem Betätigungsschlauch (42) bestehen, der einen Führungsschlauch (12) umschließt; und
die Lokalisierungsmittel des Stents aus einem Federelement (40) bestehen, das um den Schlauch (12) gewunden ist und gegenüberliegende Enden aufweist, die jeweils an den Betätigungsschlauch (16) und an das Ende des Schlauches (12) in der Nähe des Ballons (22) anstoßen oder damit verbunden sind;
woraufhin beim Gleiten der Betätigungseinrichtung (42) zu dem Ausdehnungsballon (22) hin, das Federelement (40) zusammengedrückt wird und Schleifen ausbildet, die sich seitlich von einer Achse des Ballons (22) erstrecken, und seitlich von dem darauf positionierten Stent dergestalt, dass sie einen Anschlag für das akkurate Fixieren des ausdehnbaren Stents darstellen, der auf dem Ballon angebracht ist.

## Revendications

1. Système de positionnement d'une endoprothèse, comprenant :
un ballon gonflable (22) pour dilater une endoprothèse, ledit ballon, dans son état affaissé, s'emboîtant dans et étant adapté pour transporter ladite endoprothèse dans son état pré-dilaté ; un moyen de positionnement d'endoprothèse (14) adapté pour pénétrer de manière coulissante dans un cathéter de guidage et adapté pour changer de forme avant de rentrer en contact avec la surface de la paroi intérieure d'un vaisseau sanguin principal dans la région ostiale d'un vaisseau sanguin plus petit relié audit vaisseau principal et avant la dilatation de ladite endoprothèse, et
un procédé mécanique (16) pour changer la forme dudit moyen de positionnement d'endoprothèse ; **caractérisé en ce que** :
le changement de forme permet audit moyen de positionnement de jouxter ladite surface de paroi intérieure, ce qui assure une apposition correcte entre l'endoprothèse et l'ostium dudit vaisseau sanguin plus petit ;
le procédé mécanique est constitué par un tube d'actionnement (16) entourant un tube de guidage (12) ; et
le moyen de positionnement de l'endoprothèse est sous la forme d'un manchon (18) élastomère, le côté du ballon de dilatation dudit manchon étant attaché de manière fixe au tube de guidage (12) et l'autre extrémité dudit manchon étant attaché de manière fixe au tube d'actionnement (16).

2. Système selon la revendication 1, dans lequel ledit manchon élastomère est fourni avec une pluralité de fentes longitudinales (20), dans lequel, lorsque ledit tube d'actionnement est poussé par rapport audit tube de guidage en direction dudit ballon de dilatation, le manchon élastomère se déformera en une pluralité de doigts.

3. Système tel que revendiqué dans la revendication 1, dans lequel le manchon élastomère est fourni avec une section transversale affaiblie (31) à environ la moitié de sa longueur, ladite section transversale affaiblie facilitant la déformation dudit manchon (30).

4. Système de positionnement d'endoprothèse, comprenant :
un ballon gonflable (22) pour dilater une endoprothèse, ledit ballon, dans son état affaissé, s'emboîtant dans et étant adapté pour transporter ladite endoprothèse dans son état pré-dilaté ; un moyen de positionnement d'endoprothèse (14) adapté pour pénétrer de manière coulissante dans un cathéter de guidage et adapté pour changer de forme avant de rentrer en contact avec la surface de la paroi intérieure d'un vaisseau sanguin principal dans la région ostiale d'un vaisseau sanguin plus petit relié audit vaisseau principal et avant la dilatation de ladite endoprothèse ; et un procédé mécanique (32) pour changer la forme dudit moyen de positionnement d'endoprothèse ;
**caractérisé en ce que** :
le changement de forme permet audit moyen de positionnement de jouxter ladite surface de paroi intérieure, ce qui assure une apposition correcte entre l'endoprothèse et l'ostium dudit vaisseau sanguin plus petit ;
le procédé mécanique est constitué par un tube d'actionnement (32) entourant un tube de guidage (12) ; et
le moyen de positionnement d'endoprothèse, au repos, se présente sous la forme d'une pièce de fil sensiblement en forme de U, possédant une toile et deux branches, dans lequel, lorsque ledit tube d'actionnement est poussé par rapport au tube de guidage en direction du ballon gonflable pour la dilatation de ladite endoprothèse, la pièce de fil en forme de U est déformée de manière élastique, augmentant sensiblement la longueur de ladite toile et lui permettant de ce fait de jouxter la surface de paroi intérieure dudit vaisseau sanguin principal.

5. Système selon la revendication 4, dans lequel ladite toile passe à travers un trou latéral (36) dans le tube de guidage (12) et dont les deux branches sont chacune solidaires d'un tube d'actionnement (16) entourant ledit tube de guidage (12).

6. Système de positionnement d'endoprothèse, comprenant :
un ballon gonflable (22) pour dilater une endoprothèse, ledit ballon, dans son état affaissé, s'emboîtant dans et étant adapté pour transporter ladite endoprothèse dans son état pré-dilaté ; un moyen de positionnement d'endoprothèse (14) adapté pour pénétrer de manière coulissante dans un cathéter de guidage et adapté pour changer de forme avant de rentrer en contact avec la surface de la paroi intérieure d'un vaisseau sanguin principal dans la région ostiale d'un vaisseau sanguin plus petit relié audit vaisseau principal et avant la dilatation de ladite endoprothèse ; et un procédé mécanique (42) pour changer la forme dudit moyen de positionnement d'endoprothèse ;
**caractérisé en ce que** :
le changement de forme permet audit moyen de positionnement de jouxter ladite surface de paroi intérieure, assurant ainsi l'apposition correcte entre l'endoprothèse et l'ostium dudit vaisseau sanguin plus petit ;
le procédé mécanique est constitué par un tube d'actionnement (42) entourant un tube de guidage (12) ; et
le moyen de positionnement de l'endoprothèse est un élément en ressort (40) enroulé autour du tube (12) et ayant des extrémités opposées qui, respectivement, s'adossent contre le, ou sont fixés au, tube d'actionnement (16) et à l'extrémité du tube (12) proche du ballon (22) ;
ensuite, le coulissement du déclencheur (42) vers le ballon de dilatation (22) comprime l'élément de ressort (40) et forme des boucles qui s'étendent latéralement à partir d'un axe du ballon (20) et de l'endoprothèse positionnée sur celui-ci, de manière à constituer une butée permettant de localiser avec précision l'endoprothèse dilatable montée sur le ballon.
